# EUROPÄISCHE PATENTSCHRIFT

(11) **EP 1 716 163 B1**
(45) Veröffentlichungstag und Bekanntmachung des Hinweises auf die Patenterteilung: **02.04.2008**
(21) Anmeldenummer: 05707328.0
(22) Anmeldetag: 11.02.2005
(51) Int. Cl.: C07H 15/04, A01N 25/30

(54) **VERFAHREN ZUR ALKOXYLIERUNG VON ALKYL-UND/ODER ALKENYLPOLYGLYKOSIDEN**
METHOD FOR THE ALKOXYLATION OF ALKYL AND/OR ALKENYL POLYGLYCOSIDES
PROCEDE D'ALKOXYLATION DE POLYGLYCOSIDES D'ALKYLE ET/OU D'ALCENYLE

(30) Priorität: 20.02.2004 DE 102004008302
(43) Veröffentlichungstag der Anmeldung: 02.11.2006
(60) Teilanmeldung: 07008238.3
(73) Patentinhaber: Cognis IP Management GmbH, 40589 Düsseldorf (DE)
(72) Erfinder: BEHLER, Ansgar, 46240 Bottrop (DE); CLASEN, Frank, 40721 Hilden (DE)
(74) Vertreter: Fabry, Bernd
(86) Internationale Anmeldenummer: PCT/EP2005/001376
(87) Internationale Veröffentlichungsnummer: WO 2005/087785

(56) Entgegenhaltungen:
- EP-A- 0 364 202
- WO-A-86/04899
- WO-A-99/17608
- DE-A1- 4 040 655
- US-A- 4 834 903
- US-A- 5 958 104
- US-A1- 2003 050 194

## Beschreibung

### Gebiet der Erfindung

Die Erfindung liegt auf dem Gebiet der nichtionischen Tenside und betrifft ein Verfahren zur Alkoxylierung von Alkyl- und/oder Alkenylpolyglykosiden.

### Stand der Technik

Eine Alkoxylierung von Hydroxylgruppen ist eine in der Chemie gängige Reaktion, bei der je nach eingesetztem Alkylenoxid Ethylen-, Propylen- und/oder Butylenoxid-Addukte der Hydroxylgruppen tragenden Verbindungen entstehen. Die Alkoxylierung kann auch bei komplexen chemischen Verbindungen wie Zucker- und Stärkederivaten erfolgen, sofern sie freie Hydroxylgruppen aufweisen.

Die Alkoxylierungsreaktionen derartiger komplexer Verbindungen erfolgen in der Regel bei erhöhten Temperaturen, unter Einsatz eines Katalysators und unter Ausschluss von Wasser. Gemäß der Amerikanischen Patentschrift US 3,737,426 erfolgt nach der Umsetzung von Stärke mit Ethylenglykol anschließend eine Alkoxylierung mit Ethylenoxid und/oder Propylenoxid bei etwa 170 °C unter Ausschluss von Wasser.

Die Amerikanische Patentschrift US 3,640,998 empfiehlt die Alkoxylierung bei 100 bis 200 °C und unter Einsatz von basischen Katalysatoren durchzuführen, um die Zersetzung von Stärke zu minimieren. Es handelt sich hier um eine Standardalkoxylierung, bei der stets Luftsauerstoff und Wasser vor der Alkoxylierung entfernt werden.

Schließlich sind aus der Amerikanischen Patentschrift US 4,834,903 Reaktionsmischungen von alkoxylierten Alkylmono- und Alkylpolyglykosiden bekannt, die hergestellt werden durch Alkoxylierung bei 120 bis 170 °C, unter Einsatz eines basischen Katalysators und unter im wesentlichen wasserfreien Bedingungen. In der besagten Schrift wird ausdrücklich herausgestellt, dass der Wassergehalt der Reaktionsmischung unter 5, vorzugsweise unter 1 Gew.% liegen muss.

Der Ausschluss von Wasser bei der Alkoxylierung gilt in der Fachwelt als unbedingt notwendig, da bisher davon ausgegangen wurde, dass das im Reaktionsgemisch enthaltene Wasser den Anteil an dem bei der Alkoxylierung anfallendem unerwünschtem Nebenprodukt Polyalkylenglykol bewirkt bzw. beeinflusst. Alkylpolyglykoside sind im Handel jedoch fast ausschließlich als wässrige Zubereitungen erhältlich, da Alkylpolyglykoside, insbesondere Alkylpolyglucoside, in wasserfreier Form hochviskos und kaum fließfähig sind. In der Regel weisen die handelsüblichen wässrigen Zubereitungen mindestens 10 Gew.-%, meist noch höhere Wassergehalte, auf. Sofern nun also Alkylpolyglykoside alkoxyliert werden sollten, galt es, die handelsüblichen wässrigen Zubereitungen der Alkylpolyglykoside vor der Alkoxylierung zu entwässern bzw. den Wassergehalt auf unter 5 Gew.% zu reduzieren. Die Entwässerung der Alkylpolyglykoside ist jedoch ein zeit- und kostenintensiver Prozess, der zusätzlich aufgrund der starken Schaumentwicklung während des Entwässerns technisch nur schwer realisierbar ist.

Es bestand daher die Aufgabe, ein neues Verfahren für die Alkoxylierung von Alkylpolyglykosiden zur Verfügung zu stellen, bei dem auf die Entwässerung der eingesetzten Alkylpolyglykosid-Zubereitungen verzichtet werden kann. Gleichzeitig sollte das nach dem Verfahren erhaltene Reaktionsprodukt höchstens geringe Mengen an dem unerwünschten Nebenprodukt Polyalkylenglykol aufweisen.

### Beschreibung der Erfindung

Gegenstand der Erfindung ist ein Verfahren zur Herstellung von alkoxylierten Alkyl- und/oder Alkenylpolyglykosiden durch Umsetzung von Alkylenoxiden mit Alkyl- und Alkenylpolyglykosiden der Formel (I),

**R¹O-[G]ₚ** **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, welches sich dadurch auszeichnet, dass man die Alkyl- und/oder Alkenylpolyglykoside der Formel **(I)** in Form von wässrigen Zubereitungen mit Wassergehalten von 10 bis 80 Gew.-%. - berechnet als wässrige Zubereitung - einsetzt.

### Alkyl- und/oder Alkenylpolyglykoside

Alkyl- und Alkenylpolyglykoside stellen bekannte nichtionische Tenside dar, die der Formel **(I)** folgen,

**R¹O-[G]ₚ** **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Die Alkyl- und/oder Alkenylpolyglykoside können sich von Aldosen bzw. Ketosen mit 5 oder 6 Kohlenstoffatomen, vorzugsweise der Glucose ableiten. Die bevorzugten Alkyl- und/oder Alkenylpolyglykoside sind somit Alkyl- und/oder Alkenylpolyglucoside. Die Indexzahl p in der allgemeinen Formel **(I)** gibt den Polymerisierungsgrad (DP), d. h. die Verteilung von Mono- und Polyglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkylpolyglykosid eine analytisch ermittelte rechnerische Größe, die meistens eine gebrochene Zahl darstellt. Vorzugsweise werden Alkyl- und/oder Alkenylpolyglykoside mit einem mittleren Polymerisierungsgrad p von 1,1 bis 3,0 eingesetzt. Aus anwendungstechnischer Sicht sind solche Alkyl- und/oder Alkenylpolyglykoside bevorzugt, deren Polymerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkylpolyglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkylpolyglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkylpolyglucoside auf Basis von gehärtetem C_{12/14}-Kokosalkohol mit einem DP von 1,1 bis 3.

Wesentlich im Sinne der Erfindung ist, dass die Alkyl- und/oder Alkenylpolyglykoside in Form wässriger Zubereitungen mit Wassergehalten über 5 Gew.% vorliegen und in dieser Form der Alkoxylierung zugeführt werden. Bevorzugt werden Alkyl- und/oder Alkenylpolyglykoside der Formel (I) in Form von wässrigen Zubereitungen mit Wassergehalten von 10 bis 80 Gew.-%, insbesondere von 30 bis 60 Gew.-%. - berechnet als wässrige Zubereitung - im Verfahren eingesetzt.

Für das eigentliche Alkoxylierungsverfahren hat es sich als vorteilhaft erwiesen, die Alkyl- und/oder Alkenylpolyglykoside in Form ihrer wässrigen Zubereitungen in einem Druckreaktor mit Rührer vorzulegen, den Katalysator zuzusetzen und den Autoklav vor der Reaktion gründlich mit Stickstoff zu spülen, um alle Spuren von Luftsauerstoff zu entfernen. Danach empfiehlt es sich, den Druckbehälter aufzuheizen, wobei die Alkoxylierung vorzugsweise bei Temperaturen im Bereich von 80 bis 150 und insbesondere bei 100 bis 120°C durchgeführt wird. Das Alkylenoxid, bei dem es sich um Ethylenoxid, Propylenoxid, Butylenoxid oder Mischungen hiervon handeln kann, wird vorzugsweise über einen Heber in den Reaktor eingepresst, wobei der autogene Druck bis auf maximal etwa 5 bar ansteigen kann. Vorzugsweise werden pro Mol Alkyl- und/oder Alkenylpolyglykosid durchschnittlich 0,5 bis 100, bevorzugt 0,5 bis 20 und insbesondere 1 bis 15 Mol Alkylenoxid, vorzugsweise Ethylenoxid, eingesetzt. Die Anlagerung des Alkylenoxids erfolgt dabei statistisch, d.h. bei der Alkoxyierung wird ein komplexes Gemisch unterschiedlich hoch alkoxylierter Alkyl- und/oder Alkenylpolyglykoside erhalten. Das Ende der Reaktion ist daran zu erkennen, dass der Druck im Reaktor abfällt. In der Regel liegt die Reaktionszeit zwischen 30 Minuten und 2 Stunden. Aus Sicherheitsgründen empfiehlt es sich, die Mischung nachreagieren zu lassen, vorzugsweise bei den oben genannten Temperaturen, und anschließend noch weitere 30 min bei niederen Temperaturen bis etwa 80°C, ehe der Reaktor abgekühlt und entspannt wird.

Die Alkoxylierung erfolgt in Gegenwart von Katalysatoren, vorzugsweise basischen (alkalischen) Katalysatoren wie Natriummethanolat, Natriumhydroxid und/oder Kaliumhydroxid. Besonders bevorzugt sind Natriumhydroxid und Kaliumhydroxid, die in vorteilhafter Weise in Form wässriger Lösungen eingesetzt werden, die in der Regel 40 bis 60 gew.% ig an Katalysator sind. Zweckmäßig sind Katalysatormengen von 0,1 bis 5,0 Gew.%, vorzugsweise 0,2 bis 0,6 Gew.-% - berechnet als Feststoff und bezogen auf erhaltenes Reaktionsprodukt .

### Gewerbliche Anwendbarkeit

Die nach dem erfindungsgemäßen Verfahren erhältlichen alkoxylierten Alkyl- und/oder Alkenylpolyglykosiden eignen sich insbesondere als Adjuvants in agrochemischen Formulierungen, insbesondere als Wirkverstärker für Herbizide. Unter agrochemischen Formulierungen werden hier breit alle Verbindungen verstanden, die Wirkstoffe aus der Gruppe der Fungizide, Düngemittel, Herbizide, Pestizide, Insektizide, Pflanzenstärkungsmittel oder andere Aktivsubstanzen zum Einsatz im Pflanzenbau enthalten. Besonders bevorzugt ist die Verwendung bei herbizidhaltigen Formulierungen. Vorzugsweise werden die alkoxylierten Alkyl- und/oder Alkenylpolyglykosiden als Adjuvants, insbesondere als Wirkverstärker, verwendet. Besonders herausragende Wirkungsverstärkung wird für Glyphosat beobachtet. In der Regel sind die alkoxylierten Alkyl- und/oder Alkenylpolyglykoside und die Wirkstoffe in den agrochemischen Formulierungen in Gewichtsverhältnissen von 1:40 bis 3:1, vorzugsweise von 1:20 bis 1:1 enthalten.

Bei Glyphosat handelt es sich um N-(Phosphonomethyl)glycin, C₃H₈NO₅P, MG 169,07, Schmelzpunkt 200 °C, LD₅₀ (Ratte oral) 4320 mg/kg (WHO), ein nicht-selektives systemisches Blatt-Herbizid, das vorzugsweise in Form seines Isopropylamin-Salzes zur totalen und semitotalen Bekämpfung von Ungräsern und Unkräutern, einschließlich tiefwurzelnder mehrjähriger Arten, auf allen Ackerbaukulturen, im Obst- u. Weinbau verwendet wird. Die Struktur ist wie folgt:

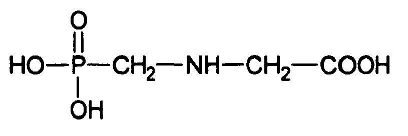

Unter Glyphosat werden alle dem Fachmann bekannten Derivate des Glyphosats verstanden, also vorzugsweise dessen Mono- oder Diethanolaminsalze des Glyphosats. Als Kationen kommen weiterhin Natrium oder Kalium in Frage. Besonders bedeutend ist das Isopropylaminsalz des Glyphosats. Weiterhin können auch beliebige Mischungen dieser Verbindungen im Rahmen der erfindungsgemäßen Verwendung eingesetzt werden.

Bei den Pestiziden, die auch in den agrochemischen Formulierungen enthalten sein können, handelt es sich vorzugsweise um öllösliche Substanzen. Es können dabei Fungizide, Herbizide, Insektizide oder deren Gemische zum Einsatz gelangen. Typische Beispiele für geeignete Fungizide sind Azoxystrobin, Benalaxyl, Carbendazim, Chlorothalonil, Cupfer, Cymoxanil, Cyproconazol, Diphenoconazol, Dinocap, Epoxiconazol, Fluazinam, Flusilazol, Flutriafol, Folpel, Fosetyl-Aluminium, Kresoxim-Methyl, Hexaconazol, Mancozeb, Metalaxyl, Metconazol, Myclobutanil, Ofurace, Phentinhydroxid, Prochloraz, Pyremethanil, Soufre, Tebucanazol, und Tetraconazol sowie deren Gemische. Als Herbizide können Alachlor, Acloniphen, Acetochlor, Amidosulfuron, Aminotriazol, Atrazin, Bentazon, Biphenox, Bromoxyl Octanoate, Bromoxynil, Clethodim, Chlodinafop-Propargyl, Chloridazon, Chlorsulfuron, Chlortoluron, Clomazon, Cycloxydim, Desmedipham, Dicamba, Dicyclofop-Methyl, Diharnstoff, Difluphenicanil, Dimithenamid, Ethofumesat, Fluazifop, Fluazifop-p-butyl, Fluorochloridon, Fluroxypyr, Glufosinat, Glyphosat, Haloxyfop-R, Ioxynil Octanoate, Isoproturon, Isoxaben, Metamitron, Metazachlor, Metolachlor, Metsulfuron-Methyl, Nicosulfuron, Notflurazon, Oryzalin, Oxadiazon, Oxyfluorphen, Paraquat, Pendimethalin, Phenmedipham, Phenoxyprop-p-Ethyl, Propaquizafop, Prosulfocarb, Quizalofop, Sulcotrion, Sulphosat, Terbutylazin, Triasulfuron, Trichlorpyr, Triflualin und Triflusulforon-Methyl einzeln oder in Abmischung eingesetzt werden. Als Insektizide kommen schließlich Biphenthrin, Carbofuran, Carbosulfan, Chlorpyriphos-Methyl, Chlorpyriphos-Ethyl, β-Cyfluthrin, λ-Cyhalothrin, Cyhexatin, Cypermethrin, Dicofol, Endosulfan, τ-Fluvalinat, α-Methrin, δ-Methrin, Phenbutatin, Pyrimicarb, Terbuphos und Tebuphenpyrad sowie deren Gemische in Betracht.

Falls gewünscht können die agrochemischen Formulierungen weitere übliche Hilfs- und Zusatzstoffe enthalten. Als fakultative Bestandteile können auch weitere Adjuvantien enthalten sein. Hierfür kommen beispielsweise nichtionogene Tenside aus mindestens einer der folgenden Gruppen in Frage:
(1) Anlagerungsprodukte von 2 bis 120 Mol Ethylenoxid und/ oder 0 bis 75 Mol Propylenoxid an lineare Fettalkohole mit 8 bis 22 C-Atomen, Fettamine, an Fettsäuren mit 8 bis 22 C-Atomen, an Alkylphenole mit 8 bis 15 C-Atomen in der Alkylgruppe und Fettaminen mit 6 bis 22 Kohlenstoffatomen;
(2) C_{12/18}-Fettsäuremono-, -di und -triester von Anlagerungsprodukten von 1 bis 120 Mol Ethylenoxid an Glycerin oder technische Oligoglycerine;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglyce-rinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

Insbesondere dann, wenn Pestizide in die Emulsionen eingearbeitet werden sollen, die bei Raumtemperatur Feststoffe darstellen, empfiehlt es sich, unpolare Lösemittel mit zu verwenden. Als weitere fakultative Komponenten kommen hierfür beispielsweise Mineralöle, Alkylaromaten und Kohlenwasserstoffe, wie sie unter der Bezeichnung Solvesso® von der Firma Exxon vertrieben werden, Fettsäureniedrigalkylester, wie z.B. die C₁-C₄-, d.h. die Methyl-, Ethyl-, Propyl- und/oder Butylester von Capronsäure, Caprylsäure, 2-Ethylhexansäure, Caprinsäure, Laurinsäure, Isotridecansäure; Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen in Frage. Des Weiteren geeignet sind pflanzliche Triglyceride, wie beispielsweise Kokosöl, Palmöl, Palmkernöl, Sonnenblumenöl, Olivenöl und dergleichen. Auch Polyethylenglykol ist ein geeignetes Lösemittel, vorzugsweise mit Molgewichten im Bereich von 90 bis 600 und vorzugsweise von 120 bis 250.

In den agrochemischen Formulierungen liegt der Wassergehalt in der Regel bei durchschnittlich 10 bis 90 und insbesondere 30 bis 60 Gew.-%. Die Applikationslösung für den eigentlichen Gebrauch enthält den eigentlichen Wirkstoff in Mengen von 0,01 bis 5 Gew.-%, vorzugsweise 0,1 bis 2,5 Gew.-% und insbesondere 0,1 bis 1,5 Gew.-%.

Die agrochemischen Formulierungen können aber auch als Konzentrate, beispielsweise mit 10 bis 90 Gew.-% Wirkstoff vertrieben werden, wobei erst vor der Anwendung die eigentliche Einsatzkonzentration durch Verdünnen eingestellt wird. Der Wassergehalt in solchen Konzentraten liegt zwischen 1 und 30 Gew.-%.

Es wurde beobachtet, dass die Kombination von Glyphosat mit den alkoxylierten Alkyl- und/oder Alkenylpolyglykosiden die Wirkung erhöht, so dass die Einsatzkonzentrationen der Wirkstoffe verringert und somit die negativen Auswirkungen des Einsatzes solcher Wirkstoffe auf die Umwelt wirksam reduziert werden können.

### Beispiele

### Eingesetzte Verbindungen und verwendete Abkürzungen

In den folgenden Beispielen wurde ein unter dem Handelsnamen Plantacare 1200^{™} der Cognis Deutschland GmbH & Co. KG handelsübliches Alkylpolyglucosid eingesetzt. Es handelt sich um eine wässrige Zubereitung mit 51,2 Gew.-% eines Alkylpolyglucosides auf Basis eines gehärteten C_{12/14}-Kokosalkohols mit einem DP von 1,4. "EO" steht für "Ethylenoxid"; "+ 5 EO" bedeutet "mit 5 Mol Ethylenoxid" alkoxyliert

### Beispiel 1

### Plantacare 1200 + 5 EO

In einem 1-1-Rührautoklaven wurden 697,0 g (entsprechend 0,8 Mol) Plantacare 1200^{™} zusammen mit 4,7 g (entsprechend 0,67 Gew.-% bezogen auf Ausgangsverbindung) einer wässrigen 50 Gew.-%igen Kaliumhydroxid-Lösung in einem Druckbehälter vorgelegt. Der Autoklav wurde verschlossen und dreimal abwechselnd mit Stickstoff gespült. Anschließend wurde bei maximal 120 °C und maximal 5 bar Druck portionsweise 178,4 g (entsprechend 4,0 Mol) Ethylenoxid eindosiert. Die Reaktionszeit betrug 1 Stunde. Nach Beendigung der Ethoxylierung wurde 1 Stunde bei 120 °C nachreagiert und 30 Minuten bei 80 °C die Apparatur evakuiert, um Reste an nicht umgesetztem Ethylenoxid zu entfernen.

Das entstandene Produkt wies folgende Qualitätskennziffern auf:
Gehalt an nicht umgesetztem Plantacare 1200^{™} (in Gew.-%): 8,1 bezogen auf Monoglucosid
Polyethylenglykolgehalt (in Gew.-%): kleiner 0,1
Wassergehalt (in Gew.-%): 34,3

### Beispiel 2

### Plantacare 1200 + 10 EO

In einem 1-1-Rührautoklaven wurden 568,0 g (entsprechend 0,69 Mol) Plantacare 1200^{™} zusammen mit 4,7 g (entsprechend 0,67 Gew.-% bezogen auf Ausgangsverbindung) einer wässrigen 50 Gew.-%igen Kaliumhydroxid-Lösung in einem Druckbehälter vorgelegt. Der Autoklav wurde verschlossen und dreimal abwechselnd mit Stickstoff gespült. Anschließend wurde bei maximal 120 °C und maximal 5 bar Druck portionsweise 304,0 g (entsprechend 6,9 Mol) Ethylenoxid eindosiert. Die Reaktionszeit betrug 1 Stunde und 25 Minuten. Nach Beendigung der Ethoxylierung wurde 1 Stunde bei 120 °C nachreagiert und 30 Minuten bei 80 °C die Apparatur evakuiert, um Reste an nicht umgesetztem Ethylenoxid zu entfernen.

Das entstandene Produkt wies folgende Qualitätskennziffern auf:
Gehalt an nicht umgesetztem Plantacare 1200^{™} (in Gew.-%): 2,2 bezogen auf Monoglucosid
Polyethylenglykolgehalt (in Gew.-%): kleiner 0,1
Wassergehalt (in Gew.-%): 24,7

## Patentansprüche

1. Verfahren zur Herstellung von alkoxylierten Alkyl- und/oder Alkenylpolyglykosiden durch Umsetzung von Alkylenoxiden mit Alkyl- und Alkenylpolyglykosiden der Formel **(I),**
**R¹O-[G]ₚ** **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, **dadurch gekennzeichnet, dass** man die Alkyl- und/oder Alkenylpolyglykoside der Formel **(I)** in Form von wässrigen Zubereitungen mit Wassergehalten von 10 bis 80 Gew.-%. - berechnet als wässrige Zubereitung - einsetzt.

2. Verfahren nach Anspruch 1, **dadurch gekennzeichnet, dass** man Alkyl- und/oder Alkenylpolyglykoside der Formel **(I)** einsetzt, in der R¹ für einen Alkylrest mit 12 bis 14 Kohlenstoffatomen steht.

3. Verfahren nach den Ansprüchen 1 und/oder 2, **dadurch gekennzeichnet, dass** man Alkyl- und/oder Alkenylpolyglykoside der Formel **(I)** einsetzt, in der p für Zahlen von 1,1 bis 3 steht.

4. Verfahren nach mindestens einem der Ansprüche 1 bis 3, **dadurch gekennzeichnet, dass** man Alkyl- und/oder Alkenylpolyglykoside der Formel **(I)** einsetzt, in der G für einen Glucoserest steht.

5. Verfahren nach mindestens einem der Ansprüche 1 bis 4, **dadurch gekennzeichnet, dass** man die Umsetzung bei Temperaturen im Bereich von 80 bis 150 °C durchführt.

6. Verfahren nach mindestens einem der Ansprüche 1 bis 5, **dadurch gekennzeichnet, dass** man die Umsetzung in Anwesenheit von 0,1 bis 5,0 Gew.-% - bezogen auf erhaltenes Reaktionsprodukt - eines basischen Katalysators durchführt.

7. Verfahren nach mindestens einem der Ansprüche 1 bis 6, **dadurch gekennzeichnet, dass** man pro Mol Alkyl- und/oder Alkenylpolyglykosid der Formel **(I)** 0,5 bis 100 Mol Alkylenoxid einsetzt.

8. Verfahren nach mindestens einem der Ansprüche 1 bis 8, **dadurch gekennzeichnet, dass** man als Alkylenoxid Ethylenoxid einsetzt.

## Claims

1. Process for the production of alkoxylated alkyl and/or alkenyl polyglycosides by reaction of alkylene oxides with alkyl and alkenyl polyglycosides corresponding to formula **(I):**
**R¹O-[G]ₚ** **(I)**
in which R¹ is an alkyl and/or alkenyl group containing 4 to 22 carbon atoms, G is a sugar unit containing 5 or 6 carbon atoms and p is a number of 1 to 10, **characterized in that** the alkyl and/or alkenyl polyglycosides corresponding to formula (I) are used in the form of water-containing preparations with water contents of 10 to 80% by weight, based on the water-containing preparation.

2. Process as claimed in claim 1, **characterized in that** alkyl and/or alkenyl polyglycosides corresponding to formula (I), in which R¹ is an alkyl group containing 12 to 14 carbon atoms, are used.

3. Process as claimed in claims 1 and/or 2, **characterized in that** alkyl and/or alkenyl polyglycosides corresponding to formula (I), in which p is a number of 1.1 to 3, are used.

4. Process as claimed in at least one of claims 1 to 3, **characterized in that** alkyl and/or alkenyl polyglycosides corresponding to formula (I), in which G is a glucose unit, are used.

5. Process as claimed in at least one of claims 1 to 4, **characterized in that** the reaction is carried out at temperatures in the range from 80 to 150°C

6. Process as claimed in at least one of claims 1 to 5, **characterized in that** the reaction is carried out in the presence of 0.1 to 5.0% by weight, based on the reaction product obtained, of a basic catalyst.

7. Process as claimed in at least one of claims 1 to 6, **characterized in that** 0.5 to 100 mol alkylene oxide is used per mol alkyl and/or alkenyl polyglycoside.

8. Process as claimed in at least one of claims 1 to 8, **characterized in that** ethylene oxide is used as the alkylene oxide.

## Revendications

1. Procédé de préparation de polyglycosides d'alkyle et/ou d'alcényle alcoxylés par réaction d'oxydes d'alkylène avec des polyglycosides d'alkyle et d'alcényle de formule (I)
R¹O([G]ₚ (I)
dans laquelle R¹ représente un radical alkyle et/ou alcényle comportant de 4 à 22 atomes de carbone, G un groupe sucre comportant 5 ou 6 atomes de carbone et p des nombres de 1 à 10,
**caractérisé en ce qu'**
on utilise les polyglycosides d'alkyle et/ou d'alcényle de formule (I) sous forme de préparations aqueuses ayant des teneurs en eau de 10 à 80 % en poids, calculées en tant que préparation aqueuse.

2. Procédé selon la revendication 1,
**caractérisé en ce qu'**
on utilise des polyglycosides d'alkyle et/ou d'alcényle de formule (I) dans laquelle R¹ représente un radical alkyle comportant de 12 à 14 atomes de carbone.

3. Procédé selon les revendications 1 et/ou 2,
**caractérisé en ce qu'**
on utilise des polyglycosides d'alkyle et/ou d'alcényle de formule (I) dans laquelle p représente des nombres de 1,1 à 3.

4. Procédé selon au moins l'une des revendications 1 à 3,
**caractérisé en ce qu'**
on utilise des polyglycosides d'alkyle et/ou d'alcényle de formule (I) dans laquelle G représente un groupe glucose.

5. Procédé selon au moins l'une des revendications 1 à 4,
**caractérisé en ce qu'**
on met en oeuvre la réaction à des températures de l'ordre de 80 à 150°C.

6. Procédé selon au moins l'une des revendications 1 à 5,
**caractérisé en ce qu'**
on met en oeuvre la réaction en présence de 0,1 à 5,0 % en poids, par rapport au produit de la réaction obtenu, d'un catalyseur basique.

7. Procédé selon au moins l'une des revendications 1 à 6,
**caractérisé en ce qu'**
on utilise de 0,5 à 100 moles d'oxyde d'alkylène par mole de polyglycoside d'alkyle et/ou d'alcényle de formule (I).

8. Procédé selon au moins l'une des revendications 1 à 8,
**caractérisé en ce qu'**
on utilise de l'oxyde d'éthylène en tant qu'oxyde d'alkylène.
